(19) Europäisches Patentamt
European Patent Office
Office européen des brevets

(11) EP 0 305 646 B1

(12) **EUROPEAN PATENT SPECIFICATION**

(45) Date of publication and mention of the grant of the patent:
**13.11.1996 Bulletin 1996/46**

(51) Int. Cl.[6]: **C07D 213/69**, A61K 31/44

(21) Application number: **88107000.7**

(22) Date of filing: **20.03.1984**

(54) **3-Hydroxypyrid-4-ones and pharmaceutical compositions**

3-Hydroxypyrid-4-one und pharmazeutische Zusammensetzungen

3-Hydroxypyrid-ones et compositions pharmaceutiques

(84) Designated Contracting States:
**BE CH DE FR GB IT LI NL SE**

(30) Priority: **24.03.1983 GB 8308054**

(43) Date of publication of application:
**08.03.1989 Bulletin 1989/10**

(62) Application number of the earlier application in accordance with Art. 76 EPC: **84301881.3**

(73) Proprietor: **BRITISH TECHNOLOGY GROUP LIMITED**
**London SE1 6BU (GB)**

(72) Inventors:
- **Hider, Robert Charles**
  **Clacton Essex (GB)**
- **Kontoghiorghes, George**
  **London N19 (GB)**
- **Silver, Jack**
  **London NW3 (GB)**

(74) Representative: **Dolan, Anthony Patrick et al**
**British Technology Group**
**101 Newington Causeway**
**London SE1 6BU (GB)**

(56) References cited:
**EP-A- 0 093 498**

- **BULLETIN OF THE CHEMICAL SOCIETY OF JAPAN, vol. 52, no. 1, January 1979, pages 111-113; K. IMAFUKU et al.: "Substituent effects on 6-substituted 3-hydroxy-1- methyl-4-pyridones"**
- **JOURNAL OF MEDICINAL CHEMISTRY, vol. 17, no. 1, January 1974, pages 1-5, American Chemical Society; L.E. HARE et al.: "Aromatic amino acid hydroxylase inhibitors. 3. In vitro inhibition by azadopamine analogs"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 305 646 B1

## Description

This invention relates to compounds for use in pharmaceutical compositions.

Certain pathological conditions such as thalassaemia, sickle cell anaemia, idiopathic haemochromatosis and aplastic anaemia are treated by regular blood transfusions. It is commonly found that such transfusions lead to a widespread iron overload, which condition can also arise through increased iron absorption by the body in certain other circumstances. Iron overload is most undesirable since, following saturation of the ferritin and transferrin in the body, deposition of iron can occur and many tissues can be adversely affected, particular toxic effects being degenerative changes in the myocardium, liver and endocrine organs. Such iron overload is most often treated by the use of desferrioxamine. However, this compound is an expensive natural product obtained by the culture of Streptomyces and, as it is susceptible to acid hydrolysis, it cannot be given orally to the patient and has to be given by a parenteral route. Since relatively large amounts of desferrioxamine may be required daily over an extended period, these disadvantages are particularly relevant and an extensive amount of research has been directed towards the development of alternative drugs. However, work has been concentrated on three major classes of iron chelating agents or siderophores, namely hydroxamates, ethylenediamine tetra-acetic acid (EDTA) analogues and catechols. The hydroxamates generally suffer from the same defects as desferrioxamine, being expensive and acid labile, whilst the other two classes are ineffective at removing iron from intracellular sites. Moreover, some catechol derivatives are retained by the liver and spleen and EDTA analogues possess a high affinity for calcium and so are also likely to have associated toxicity problems.

We have accordingly studied the iron chelating ability of a wide range of compounds and in UK patent application 8308056, published as GB-A-2,118,176 (corresponding to EP-A-0093498) we describe a group of compounds which we have identified as being of particular use for the treatment of conditions involving iron overload. These compounds consist of 3-hydroxypyrid-2- and -4-ones in which the nitrogen atom and optionally one or more of the carbon atoms of the ring are substituted by an aliphatic hydrocarbon group, particularly of 1 to 6 carbon atoms. We have now found that 3-hydroxypyrid-4-ones containing other non-ionisable ring substituents are also of particular value in the treatment of such conditions.

Hare et al., Journal of Medicinal Chemistry 1974, 17, 1, describe the activity of certain 1-aminoalkyl-3-hydroxypyrid-4-ones as aromatic amino acid hydroxylase inhibitors but make no reference to the use of hydroxypyridones in removing toxic concentrations of metals from the body.

6-Chloromethyl-3-hydroxy-1-methypyrid-4-one is described by Imafuku et al., Bulletin of the Chemical Society of Japan 1979, 52, 111; 3-hydroxy-6-hydroxymethyl-1-methypyrid-4-one is described by the same authors and also by Imafuku et al., ibid, 1979, 52 107 and by Berson et al., Journal of the American Chemical Society, 1956, 78, 622; 1-ethyl-3-hydroxy-6-hydroxymethylpyrid-4-one is described by Imafuku et al., ibid, 1979, 52, 107; 3-hydroxy-1-(2'-hydroxyethyl)-2-methylpyrid-4-one is described by Yasue et al., Yagugaku Zasshi, 1970, 90, 1222; and 1-methoxycarbonylmethyl-3-hydroxy-2-methyl-pyrid-4-one is described by Severin et al., Zeitschrift fur Lebensmittel-Untersuchung und-Forschung, 1976, 161, 119. None of these papers refers to any therapeutic use of these compounds.

Accordingly the present invention comprises a compound of the formula (1)

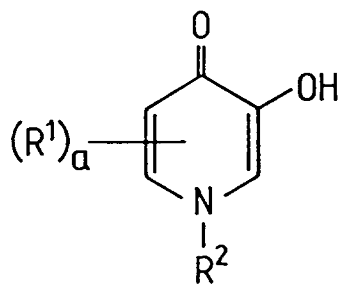

(1)

in which each $R^1$ separately is a $C_{1-6}$ aliphatic hydrocarbon group or a $C_{1-8}$ aliphatic hydrocarbon group substituted by a formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen or hydroxy group, $R^2$ is a $C_{1-6}$ aliphatic hydrocarbon group, a formyl or ($C_{1-4}$ alkyl)-carbonyl group, or a $C_{1-8}$ aliphatic hydrocarbon group substituted by one or more substituents selected from formyl, ($C_{1-4}$ alkyl)-carbonyl, carbamoyl, sulphamoyl, mono- and di-$C_{1-6}$ aliphatic hydrocarbyl N-substituted carbamoyl and N-substituted sulphamoyl, formylamino, ($C_{1-6}$ aliphatic hydrocarbyl)-carbonylamino, ($C_{1-6}$ aliphatic hydrocarbyl)-sulphonylamino, mono-$C_{1-6}$ aliphatic hydrocarbyl N-substituted formylamino, N-substituted

($C_{1-6}$ aliphatic hydrocarbyl)-carbonylamino and N-substituted ($C_{1-6}$ aliphatic hydrocarbyl)- sulphonylamino, formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen and hydroxy groups, and a is 1, 2 or 3, but excluding compounds in which each $R^1$ and $R^2$ is selected from $C_{1-6}$ aliphatic hydrocarbon groups and those in which the grouping >N-$R^2$ contains either a group

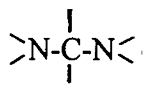

or a group

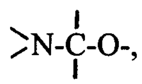

or a physiologically acceptable salt thereof, for use in therapy.

The ability of both the free compound and its iron complex to permeate membranes is important in the context of the treatment of iron overload and it is also desirable for both to possess some degree of water solubililty. A good indication of the physical properties of a compound and its iron complex in this respect is provided by the value of the partition coefficient ($K_{part}$) obtained on partition between n-octanol and tris hydrochloride (20 mM, pH 7.4; tris representing 2-amino-2-hydroxymethylpropane 1,3-diol) at 20°C and expressed as the ratio (concentration in organic phase)/(concentration in aqueous phase). Preferred compounds show a value of $K_{part}$ for the free compound of above 0.02 or 0.05 but less than 3.0, especially of above 0.2 but less than 1.0, together with a value of $K_{part}$ for the 3:1 hydroxypyridone:iron(III) complex of above 0.02 but less than 6.0, especially of above 0.2 but less than 1.0. The following comments upon preferences among the groups used for replacement of hydrogen atoms attached to nitrogen or carbon atoms of the pyridone ring are directed towards the use of compounds having partition coefficients in the free and complexed state which lie in these preferred ranges. For examples of measured partition coefficients of specific compounds reference should be made to Table 1 of Example 12.

Compounds of particular interest are those in which the hydrogen atom attached to the nitrogen atom of the pyridone ring is replaced by a group other than an aliphatic hydrocarbon group, although the invention does of course extend to compounds in which this hydrogen atom is replaced by an aliphatic hydrocarbon group and a hydrogen atom attached to a carbon atom of the ring is replaced by another type of group. Furthermore, although compounds having a group other than an aliphatic hydrocarbon group attached to the ring nitrogen atom may also have the hydrogen atom of one or more ring carbon atoms replaced by the same or a different group which is also other than an aliphatic hydrocarbon group, it is preferred either that the ring carbon atoms in such compounds are unsubstituted or that substitution is limited to aliphatic hydrocarbon groups.

More than one of the ring carbon atoms of the 3-hydroxypyrid-4-one may be substituted, for example two of such atoms, either by the same substituent group or by different substituent groups, for example by an aliphatic hydrocarbon group and by another type of substituent, although compounds in which none or only one of the ring carbon atoms are substituted, for example by an aliphatic hydrocarbon group, are preferred. Thus, for example, substitution of the carbon atoms is of interest at the 6- or particularly the 2-position. Particularly when the ring carbon atoms are substituted by the larger groups, however, there may be an advantage in avoiding substitution on a carbon alpha to the

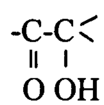

system.
This system is involved in the complexing with iron and the close proximity of one of the larger aliphatic hydrocarbon groups may lead to steric effects which inhibit complex formation.

Where reference is made to a ring nitrogen or carbon atom being substituted by an aliphatic hydrocarbon group it will be appreciated that the term aliphatic hydrocarbon group is used herein to include both acyclic and cyclic groups which may be unsaturated or saturated, the acyclic groups having a branched or especially a straight chain. Groups of from 1 to 6 carbon atoms, particularly of 1 to 4 and especially of 1 to 3 carbon atoms, are of most interest. Saturated aliphatic hydrocarbon groups are preferred, these being either cyclic groups such as the cycloalkyl groups cyclopropyl

and especially cyclohexyl or, more particularly, acyclic groups such as the alkyl groups methyl, ethyl, n-propyl and isopropyl. Where the ring carbon atoms are substituted by an aliphatic hydrocarbon group or groups these are preferably methyl but in the case of a group substituting the nitrogen atom larger groups may more often be utilised with particular advantage.

In the case of substituted aliphatic hydrocarbon groups, the preferences as to the nature of the aliphatic hydrocarbon group are broadly as expressed above but the preferences as to size are somewhat different. Firstly, in the case of such groups attached to nitrogen, groups containing only 1 carbon atom are of less interest, and indeed are excluded for certain substituents, such as hydroxy groups, since compounds containing such substituted groups may be difficult to prepare because of a relative lack of stability of the systems

-N.C.O- and -N.C.N-.

Furthermore, in the case of substituted aliphatic hydrocarbon groups attached either to a nitrogen atom or to a carbon atom, a substituted methyl group containing a hydrophilic substituent may cause the compound to be of too hydrophilic a character unless the effect of this group is balanced by that of another, more hydrophobic, group. For this reason aliphatic hydrocarbon groups of 2 or especially 3 or more carbon atoms are often preferred in this context, particularly where the substituent is a hydroxy group, although groups of 1 or 2 carbon atoms may be very suitable in certain cases, for example where the substituent is an amide group, particularly an N-substituted one. It will be seen that the range of size of aliphatic hydrocarbon groups which are substituted is greater than for unsubstituted groups, being 1 to 8 carbon atoms, although those of 1 to 6 carbon atoms are preferred, especially those of 3, 4 or 5 carbon atoms when the substituted group contains no additional hydrophobic group.

In general, the circumstances in which the smaller substituted aliphatic hydrocarbon groups are of interest occur either when the substituent group involved is not of a particularly hydrophilic character or when it is of such a character but an additional group, such as a larger aliphatic hydrocarbon group of, for example 3 or more carbon atoms is present to provide a balance to the hydrophilic character. Such a larger group may be present in the same substituted aliphatic hydrocarbon group, as is the case with the amide-containing groups referred to above, or separately. In particular, when both the ring nitrogen atom and one or more of the ring carbon atoms carry a substituted aliphatic hydrocarbon group, it may be suitable for one of the substituted aliphatic hydrocarbon groups, for example that carried by the nitrogen atom, to be of a size as discussed above, for example of 3 or 4 to 5 or 6 carbon atoms, and for the other or others to be smaller, for example of 2 or particularly 1 carbon atom. Thus, when the nitrogen atom carries either a substituted aliphatic hydrocarbon group or an acyl group and one or more carbon atoms, for example that at the 6-position, are also substituted, then as an alternative or in addition to such C-substitution being effected by an aliphatic hydrocarbon group, it may conveniently be effected by a substituted methyl group. It will be appreciated that substitution at the nitrogen atom of the compounds is of particular interest in the present invention and that it is preferred to dictate the hydrophilic/hydrophobic balance in the molecule primarily through such N-substitution. It is more usually the case therefore that, when the compounds are C-substituted but the preferred form of C-substitution involving aliphatic hydrocarbon groups is not present, the C-substituent or substituents incorporate substituted aliphatic hydrocarbon groups which are such that preferences as to the size and nature of these groups are broadly as expressed for the unsubstituted groups, for example being substituted alkyl groups of 1 to 3 carbon atoms and particularly substituted methyl groups such as chloromethyl, ethoxymethyl, and especially hydroxymethyl.

Substituted aliphatic hydrocarbon groups attached to the nitrogen atom of compounds of use in the present invention may contain more than one substituent, for example two substituents of a different type. One example of such a multiply substituted aliphatic hydrocarbon group occurs where the group is substituted both by a sulphonic or particularly a carboxylic acid ester group and by a sulphonic or particularly a carboxylic acid amide group. One potential drawback with such multiply substituted groups, however, is their increased size and it is the case, therefore, that groups containing one substituent, often terminally substituted on the aliphatic hydrocarbon group, are more commonly employed.

As regards the substituent groups present in the compounds of use in the present invention, an aliphatic acyl group may contain a sulphonyl or carbonyl group. The latter type are however preferred and although the acyl group may be a formyl group, alkylcarbonyl and cycloalkylcarbonyl groups are of most interest. Such acyl groups may be of 2 to 4 or 5 carbon atoms, and particularly may contain alkyl and cycloalkyl groups of the type described above as being preferred as an aliphatic hydrocarbon group substituent at a ring nitrogen or carbon atom, being for example $-COCH_2CH_3$ or especially $-COCH_3$.

Amide substituents may contain a sulphonyl or a carbonyl group. The latter type are, however, of most interest and the further discussion will therefore refer to them although it applies equally to the sulphonyl type. The amide substituent may be unsubstituted, being a carbamoyl group $-CONH_2$, or may be a carbamoyl group in which the nitrogen atom

is mono- or di-substituted by a $C_{1-6}$ aliphatic hydrocarbon group, for example an alkyl or cycloalkyl group such as is described above as a substituent at a ring nitrogen or carbon atom, for example being a group $-CONHCH_3$, etc. Alternatively, the

$$-CO.\overset{|}{N}-$$

grouping of the amide substituent may be arranged in the opposite sense to that in an N-substituent, for example, the nitrogen atom of the amide grouping is linked to the aliphatic hydrocarbon group which is attached to the nitrogen atom of the ring, the carbonyl group being attached to a $C_{1-6}$ aliphatic hydrocarbon group, for example an alkyl or cycloalkyl group such as is described above is a substituent at a ring nitrogen or carbon atom, or in the case of a carboxylic acid amide but not in that of a sulphonic acid amide, to hydrogen. In the case of this latter form of amide group, the amide nitrogen atom may carry a hydrogen atom or be mono-substituted as discussed for amide substituents of the first mentioned form, that form of amide substituent being the one of particular interest.

Ester substituents may contain a sulphonyloxy or preferably a carbonyloxy group and may be arranged in either sense, i.e. with a carboxylic acid ester the group -CO.O- may have either the carbonyl group or the oxy group linked to the carbon atom of the ring or the nitrogen atom of the ring through the aliphatic hydrocarbon group on which the ester group is substituted. The other group of oxy and carbonyl will be linked to an aliphatic hydrocarbon group forming part of the ester group or, in the case where this is a carbonyl group may alternatively be linked to hydrogen (this possibility does not apply in the case of sulphonic acid esters). Once again, preferred aliphatic hydrocarbon groups contained by the ester group are those described above as substituents in relation to substitution on a ring nitrogen or carbon atom. In the case of N-substituents, ester groups in which the carbonyl or sulphonyl group is linked to the aliphatic hydrocarbon group which is in turn attached to the ring are preferred, for example the groups $-CH_2CO_2CH_3$ and $-CH_2CO_2C_2H_5$. In the case of C-substituents the reverse is true and there is a strong preference for the oxy group to be attached to the aliphatic hydrocarbon group which is in turn attached to the ring, for example as in the groups $-CH_2O.COCH_3$ and $-CH_2O.COC_2H_5$. Halogen substituents may conveniently be iodo, fluoro, bromo or especially chloro.

Among the substituents on the ring nitrogen atom of some particular interest are aliphatic acyl groups and aliphatic hydrocarbon groups substituted by acyl, amide, carboxy, aliphatic ester and hydroxy groups, for example the groups $-COR_1$, $-(CH_2)_n-COXR_2$ and $-(CH_2)_m-OH$, and to a lesser extent the more complex type of group $-(CH_2)_nCH(COY)NHCOR_2$, in which $R_1$ is an alkyl or cycloalkyl group, for example methyl, ethyl or n-propyl, $R_2$ is hydrogen or an alkyl or cycloalkyl group, for example methyl, ethyl, n-propyl, isopropyl or butyl, X is an imino or, when $R_2$ is not hydrogen, an oxy group, Y is $OR_1$ or $NR_2$, n is an integer from 1 to 4 or 6, particularly 2, 3 or 4 and m is an integer from 2 to 4 or 6, particularly 3, 4 or 5. It will be appreciated that there is an inter-relation between the preferred values of n and $R_2$ so that the groups having the higher values of n tend to have the lower values of $R_2$, and vice versa, so that in groups $-(CH_2)_n-COXR_2$, for example, $-(CH_2)_n-$ and $R_2$ may conveniently together contain 3 to 7 carbon atoms, especially 4 to 6 carbon atoms. Also of interest as N-substituents in the case where a ring carbon atom is substituted by other than an aliphatic hydrocarbon group are aliphatic hydrocarbon groups, for example a group $R_1$ such as those described above.

Among substituents on the ring carbon atoms which are of some particular interest are aliphatic hydrocarbon groups and such groups substituted by a halogen or especially a hydroxy group, for example the groups $-CH_2Cl$ and $-CH_2OH$ and groups $R_1$ such as those described above.

The N-substituents described above may be present on the nitrogen atom of various 3-hydroxypyrid-4-ones, in particular 3-hydroxypyrid-4-one, 2-alkyl (e.g methyl and ethyl)-3-hydroxypyrid-4-ones, 6-alkyl-(e.g. methyl)-3-hydroxypyrid-4-ones, 2,6-dialkyl(e.g. dimethyl)-3-hydroxypyrid-4-ones and 3-hydroxy-6-hydroxymethylpyrid-4-one. N-substituent groups of the aliphatic acyl and amide-, ester- and hydroxy-substituted aliphatic hydrocarbon group type are of especial interest.

The compounds may, if desired, be used in the form of salts formed at the hydroxy group thereof through its conversion to the anion

$$(OH \rightarrow O^-)$$

and containing a physiologically acceptable cation, for example the cation of an alkali metal such as sodium, quaternary ammonium ions or protonated amines such as the cation derived from tris (tris represents 2-amino-2-hydroxymethyl propane 1,3-diol). Salt formation may be advantageous in increasing the water solubility of a compound but, in general, the use of the compounds themselves rather than their salts, is preferred.

Specific examples of N-substituted 3-hydroxypyrid-4-ones lacking C-substitution other than by an aliphatic hydrocarbon group are the following compounds, with $R_1$ and $R_2$ being as defined above, X being oxy or imino [but not oxy in formulae (III) and (IV) when $R_2$ is hydrogen] and R being hydrogen, ethyl or especially methyl. Compound (III) in

which X is an imino group may be mentioned particularly.

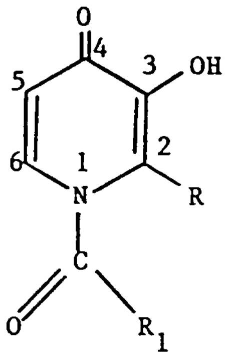

(I)

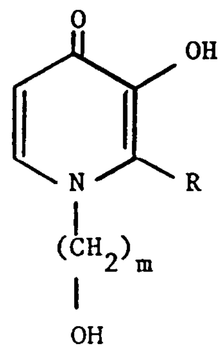

(II)

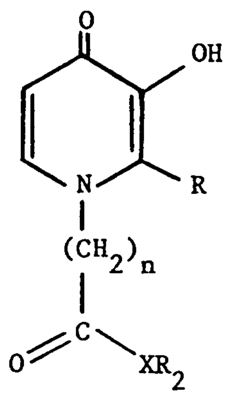

(III)

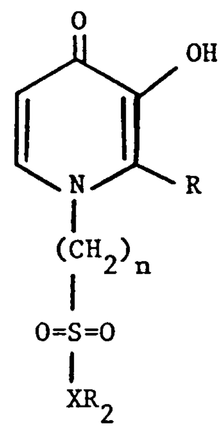

(IV)

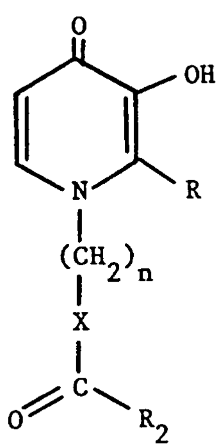

(V)

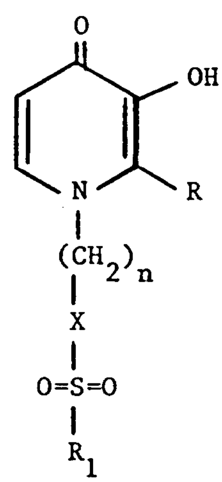

(VI)

Examples of pyridones containing C-substituents other than aliphatic hydrocarbon groups and N-substituents which are aliphatic hydrocarbon groups are the 1-alkyl-6-halomethyl-3-hydroxypyrid-4-ones and the 1-alkyl-3-hydroxy-6-hydroxymethylpyrid-4-ones, specific examples of which are the following compounds in which the various symbols

are as defined above and Hal represents a halogen group.

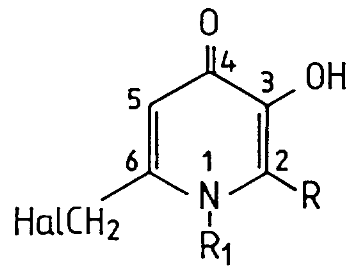

(VII)

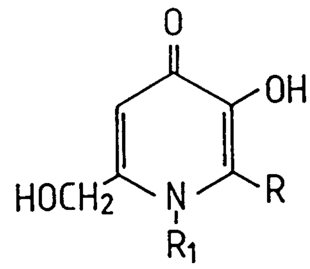

(VIII)

Most of the compounds of formula (1) are novel except as described hereinbefore.

The present invention thus also includes as compounds, per se, a compound of the formula (1)

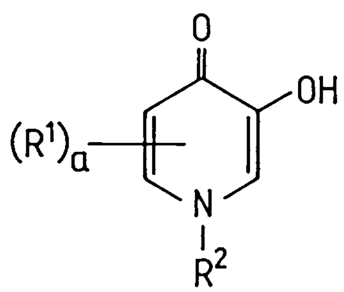

(1)

in which each $R^1$ separately is a $C_{1-6}$ aliphatic hydrocarbon group or a $C_{1-8}$ aliphatic hydrocarbon group substituted by a formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen or hydroxy group, $R^2$ is a $C_{1-6}$ aliphatic hydrocarbon group, a formyl or ($C_{1-4}$ alkyl)-carbonyl group, or a $C_{1-8}$ aliphatic hydrocarbon group substituted by one or more substituents selected from formyl, ($C_{1-4}$ alkyl)-carbonyl, carbamoyl, sulphamoyl, mono- and di-$C_{1-6}$ aliphatic hydrocarbyl N-substituted carbamoyl and N-substituted sulphamoyl, formylamino, ($C_{1-6}$ aliphatic hydrocarbyl)-carbonylamino, ($C_{1-6}$ aliphatic hydrocarbyl)-sulphonylamino, mono-$C_{1-6}$ aliphatic hydrocarbyl N-substituted formylamino, N-substituted ($C_{1-6}$ aliphatic hydrocarbyl)-carbonylamino and N-substituted ($C_{1-6}$ aliphatic hydrocarbyl)-sulphonylamino, formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen and hydroxy groups, and a is 1, 2 or 3; but excluding compounds in which each $R^1$ and $R^2$ is selected from $C_{1-6}$ aliphatic hydrocarbon groups, compounds in which the grouping $>N-R^2$ contains either a group

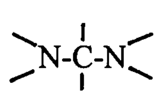

or a group

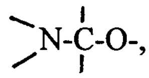

and compounds in which $(R^1)_a$ is a 6-chloromethyl group and $R^2$ is a methyl group, $(R^1)_a$ is a 6-hydroxymethyl group and $R^2$ is a methyl or ethyl group, or $(R^1)_a$ is a 2-methyl group and $R^2$ is a 2-hydroxyethyl or methoxycarbonylmethyl group; or a physiologically acceptable salt thereof.

The 3-hydroxypyrid-4-ones may conveniently be prepared from 3,4-dihydroxypyridines by similar procedures to those given in UK patent application 84071180, published as GB 2,136,806A (corresponding European patent application A-0120670) for the preparation of the 3-hydroxypyrid-2-ones from 2,3-dihydroxypyridines. Preferably, however, they are prepared from the more readily accessible corresponding 3-hydroxy-4-pyrone or a 3-hydroxy-4-pyrone containing groups convertible to the C-substituent present in the desired pyridone, for example by the reaction

$$-CH_2OH \rightarrow -CH_2Hal.$$

Thus, the 3-hydroxy-4-pyrone may conveniently be converted to the 3-hydroxypyrid-4-one through protection of the hydroxy group, for example as an ether group such as a benzyloxy group, and reaction of the protected compound with a compound $R'NH_2$, in which R' represents the group $R^2$ present on the nitrogen atom of the desired 3-hydroxypyrid-4-one or a group convertible thereto, in the presence of a base, for example an alkali metal hydroxide such as sodium hydroxide. The hydroxy protecting group is then removed and any other modifications of the C-substituents effected. In particular $R'NH_2$ may for example represent an amino substituted aliphatic hydrocarbon or a hydroxyamine, such as 2-hydroxyethylamine or 3-hydroxypropylamine. The hydroxy group in such an N-substituent may of course be converted to an ester group and N-substituent groups containing an amino or carboxy (or sulpho) group can be used as precursors for N-substituent groups containing amide and ester groups, respectively.

An alternative procedure involves the use of a 2-aliphatic acyl 3-hydroxyfuran which may be reacted with a compound $R'NH_2$, by analogy with the reaction of 2-acetyl-3-hydroxyfuran with the sodium salt of glycine to form 1-carboxymethyl-3-hydroxy-2-methylpyrid-4-one which is described by Severin, ibid.

An example of the modification of C-substituents after formation of the pyridone ring arises in the case where it is appropriate for substituents on carbon atoms of the ring to be protected whilst substitution is effected at the nitrogen atom of the ring. Thus, for example, where a hydroxymethyl group is present on the ring as well as the 3-hydroxy group, both hydroxy groups in a 3-hydroxy-4-pyrone may be protected as described above. Moreover, N-acylated pyrid-4-ones may be prepared by direct acylation but it is preferred to protect the 3-hydroxy group, for example as an ether group such as in a benzyloxy group, and to remove this protecting group after the N-acylation has been effected. A similar form of protection of the 3-hydroxy group is suitable where a 6-hydroxyalkyl group is present which is being modified, for example to give a 6-haloalkyl group.

It will be appreciated from the foregoing that in many cases the final stage in the preparation of the compound comprises deprotecting the hydroxy group of a pyridone having similar ring carbon atom substituents but with the 3-hydroxy group in protected form and either the same ring nitrogen atom substituent or one convertible thereto and, where applicable, converting the N-substituent to that present in said compound and/or, optionally, converting the compound to a physiologically acceptable salt thereof.

The compounds may be converted to salts such as those formed between a physiologically acceptable cation and the anion produced by the loss of the hydroxy group proton by reaction of the compound with the appropriate base or acid according to standard procedures.

In general, it is preferred that the compounds are isolated in substantially pure form, i.e. substantially free from by-products of manufacture.

It will be appreciated that these are not the only routes available to these compounds and that various alternatives may be used as will be apparent to those skilled in the art, as will be the routes to the various intermediates required such as C-substituted 3,4-dihydroxypyridines and 3-hydroxy-4- pyrones.

Moreover, it will be appreciated that certain of the compounds may be converted in vivo to other compounds which will be involved in the metal binding activity observed in vivo. This will be true, for example, of compounds containing ester groups which are likely to be converted to carboxy groups when the compounds are administered orally.

The compounds may be formulated for use as pharmaceuticals for veterinary, for example in an avian or especially a mammalian context, or particularly human use by a variety of methods. For instance, they may be applied as an aqueous, oily or emulsified composition incorporating a liquid diluent which most usually will be employed for parenteral administration and therefore will be sterile and pyrogen free. However, it will be appreciated from the foregoing discussion in relation to desferrioxamine that oral administration is to be preferred and the compounds of the present invention may be given by such a route. Although compositions incorporating a liquid diluent may be used for oral administration, it is preferred, particularly in humans, to use compositions incorporating a solid carrier, for example a conventional solid

carrier material such as starch, lactose, dextrin or magnesium stearate. Compositions comprising a diluent of water and/or an organic solvent which are non-sterile are therefore of less interest. Such solid compositions may conveniently be of a formed type, for example as tablets, capsules (including spansules), etc.

Other forms of administration than by injection or through the oral route may also be considered in both human and veterinary contexts, for example the use of suppositories for human administration.

Compositions may be formulated in unit dosage form. i.e. in the form of discrete portions each comprising a unit dose, or a multiple or sub-multiple of a unit dose. Whilst the dosage of active compound given will depend on various factors, including the particular compound which is employed in the composition, it may be stated by way of guidance that satisfactory control of the amount of iron present in the human body will often be achieved using a daily dosage of about 0.1 g to 5 g, particularly of about 0.5 g to 2 g, veterinary doses being on a similar g/kg body weight ratio. However, it will be appreciated that it may be appropriate under certain circumstances to give daily dosages either below or above these levels. Where desired, more than one compound according to the present invention may be administered in the pharmaceutical composition or, indeed, other active compounds may be included in the composition.

It has never before been appreciated that compounds such as those described herein might be used in a pharmaceutical context, and with real advantage. We have found that the 3-hydroxypyrid-4-ones described above are particularly suited to the removal of iron from patients having an iron overload. The compounds form neutral 3:1 iron complexes at most physiological pH values, and have the advantage that they do not co-ordinate calcium or magnesium. Both the compounds and their complexes will partition into n-octanol indicating that they will permeate biological membranes, this property being confirmed in practice by tests of the ability of the $^{59}$Fe labelled iron complexes to permeate erythrocytes.

The 3-hydroxypyrid-4-ones possess a high affinity for iron (III), as evidenced by log $K_{sol}$ values {log $K_{sol}$ is defined as being equal to log $\beta_{Fe(L)n}$ + 21 - [p$K_{sp}$ + n log $a_L$(H+) + m log $a_L$ (Ca++)] where log $\beta_{Fe(L)n}$ is the cumulative affinity constant of the ligand in question for iron (III), p$K_{sp}$ is the negative logarithm of the solubility product for $Fe(OH)_3$ and has a value of 39, n and m are the number of hydrogen and calcium ions, respectively, which are bound to the ligand, and $a_L$(H+) and $a_L$(Ca++) are the affinities of the ligand for hydrogen ions and calcium ions, respectively}. In order to solubilise iron (III) hydroxide, log $K_{sol}$ must be greater than 0 and in order to remove iron from transferrin, log $K_{sol}$ should be in excess of 6.0. The log $K_{sol}$ values for 1,2-dimethyl-3-hydroxypyrid-4-one, by way of example, is 9.5, thus comparing favourably with those of the bidentate hydroxamates at about 4.0, of catechols at about 8.0, of desferrioxamine at 6.0, and of diethylenetriamine penta-acetic acid (DTPA) at 2.0. Moreover, the ability of the compounds to remove iron efficiently has been confirmed both by in vitro test and also by in vivo tests in mice. It is particularly significant that these latter tests are successful whether the compound is given intraperitoneally or orally by stomach tube, the compounds generally either being stable under acidic conditions or being converted thereby to acid stable active compounds. Oral activity is not generally present among the other types of compound previously suggested for use as iron co-ordinating drugs and although certain EDTA analogues do show such activity, they possess drawbacks for pharmaceutical use.

In addition to the use described hereinbefore for the treatment of general iron overload, the 3-hydroxypyrid-4-ones described herein are also of interest for use in certain pathological conditions where there may be an excess of iron deposited at certain sites even though the patient does not exhibit a general iron overload, this being the case, for example, in certain arthritic and cancerous conditions. Indeed in some patients having such conditions, the patient may exhibit an overall anaemia and in the aforementioned EP A-0120670 the use is described of the metal-free compounds of the present invention in conjunction with an iron complex for the treatment of such patients. Thus, a mixture of an iron complex of a 3-hydroxypyrid-4-one as described herein together with one of the 3-hydroxypyrid-4-ones in metal-free form will have the effect of remedying the overall anaemia through the action of the iron complex whilst the metal-free compound will act to remove iron from pathological to physiological sites.

The present invention thus includes the use of a compound of formula (1) as described hereinbefore for the manufacture of a medicament for use in effecting a reduction in toxic levels of iron in a patient's body.

Although the major use of the compounds is in the removal of iron, they are also of potential interest for the removal of some other metals present in the body in deleterious amounts, for example copper, plutonium and other related transuranic metals, and especially aluminium.

Uses of the compounds of the present invention for combination with metals other than iron may extend to the treatment of body fluids outside the body or even to quite other contexts than the treatment of patients. One particular area of some interest involves the treatment of patients on haemodialysis who may show a dangerous build up of aluminium in the body.

This invention is illustrated by the following Examples. Further details relevant to the synthesis of the 3-hydroxypyrid-4-ones are provided in relation to the synthesis of the related 3-hydroxypyrid-2-ones in the aforementioned EP A-0120670, covering iron complexes of the 3-hydroxypyrid-2- and -4-ones for use in the treatment of iron deficiency anaemia.

EXAMPLES

Example 1

The preparation of 1-acetyl-3-hydroxy-2-methylpyrid-4-one 3-Benzyloxy-2-methyl-4-pyrone

3-Hydroxy-2-methyl-4-pyrone (22.2 g) in methanol (225 ml) is added to aqueous sodium hydroxide (25 ml $H_2O$ containing 7.5 g NaOH). Benzyl chloride (25.5 g) is added and the mixture is refluxed for 6 hours and is then allowed to cool overnight. The bulk of the methanol is removed under vacuum and the residue is treated with water (50 ml). The mixture is extracted into dichloromethane (3 x 25 ml). The extracts are combined, washed with 5% w/v NaOH (2 x 25 ml), then water (2 x 25 ml) and dried over magnesium sulphate. Evaporation of the solvent gives crude 3-benzyloxy-2- methyl-4-pyrone (35 g, 92%) which is purified by distillation in nitrogen under reduced pressure to yield a colourless oil (28 g) of b.p. 148°C/0.2 mm.

3-Benzyloxy-2-methylpyrid-4-one

3-Benzyloxy-2-methyl-4-pyrone (20 g), concentrated (s.g. 0.880) ammonia (200 ml) and ethanol (100 ml) are mixed and kept at room temperature for 3 days. The solvent and excess ammonia are then removed by rotary evaporation to yield an oil which on trituration with acetone gives 3-benzyloxy-2-methylpyrid-4-one as white crystals m.p. 162-163°C.

1-Acetyl-3-benzyloxy-2-methylpyrid-4-one

3-Benzyloxy-2-methylpyrid-4-one (4 g) is dissolved in dry acetone (100 ml). Acetylbromide (3 g) and triethylamine (2.7 g) are added and the resulting mixture is mechanically stirred overnight. The mixture is then filtered and the filtrate is rotary evaporated to dryness. The residue is dissolved in methylene chloride and washed with dilute hydrochloric acid (pH 3.0), then twice with water, and is dried over $Na_2SO_4$ and rotary evaporated to yield a solid residue. Recrystallisation of this residue from an ethylacetate/hexane mixture gives 1-acetyl-3-benzyloxy-2-methylpyrid-4-one as an oil (3.1 g).

1-Acetyl-3-hydroxy-2-methylpyrid-4-one

1-Acetyl-3-benzyloxy-2-methylpyrid-4-one (2 g) is treated with 45% w/v HBr-acetic acid (10 ml) for 1 hour at 100°C. The solution is rotary evaporated to dryness at 70°C and triturated with an ethyl acetate/methanol (20.1 v/v) mixture. On standing overnight at 4°C pale brown crystals are deposited (1.2 g) and these are recrystallised from an ethyl acetate/methanol mixture to yield 1-acetyl-3-hydroxy-2-methylpyrid-4-one as white crystals, m.p. 152-160°C; $\nu_{max}$ (nujol) 1630, 1680 $cm^{-1}$; $\delta(d_6DMSO)$, 2.2 (s, 3H), 2.5 (s, 3H), 7.25 (d, 1H), 8.15 (d, 1H).

Example 2

The preparation of 3-hydroxy-1-(2'-hydroxyethyl)-2-methylpyrid-4-one 3-Benzyloxy-1-(2'-hydroxyethyl)-2-methylpyrid-4-one

3-Benzyloxy-2-methyl-4-pyrone (4.8 g), prepared as described under Example 1, and 2-hydroxyethylamine (1.22 g), are dissolved in water (220 ml) and ethanol (100 ml) containing sodium hydroxide (2 g) is added. The mixture is stirred at room temperature for 6 days and is then acidified with concentrated hydrochloric acid to pH 2, and evaporated to dryness. The resulting colourless solid is washed with water and extracted into chloroform (2 x 50 ml). The chloroform extracts are combined, dried over magnesium sulphate, and evaporated to yield 3-benzyloxy-1-(2'-hydroxyethyl)-2-methylpyrid-4-one, as a white solid (3.4 g), m.p. 198-199°C.

3-Hydroxy-1-(2'-hydroxyethyl)-2-methylpyrid-4-one

3-Benzyloxy-1-(2'-hydroxyethyl)-2-methylpyrid-4-one (2 g) is added to concentrated hydrobromic acid (10 ml) and the mixture is heated on a steam bath for 30 minutes. The resultant product is then recrystallised from water to yield 3-hydroxy-1-(2'-hydroxyethyl)-2-methylpyrid-4-one as white crystals (0.8 g), m.p. 164-165°C; $\nu_{max}$ (nujol) 1630, 3150, 3350 $cm^{-1}$; $\delta(d_6DMSO)$, 2.5 (s, 3H), 3.7 (t, 2H), 4.35 (t, 2H), 7.25 (1H), 8.15 (d, 1H).

Example 3

The preparation of 3-hydroxy-1-(3'-hydroxypropyl)-2-methylpyrid-4-one

3-Benzyloxy-2-methyl-4-pyrone, prepared as described under Example 1, is reacted with 3-hydroxypropylamine under substantially similar conditions to those described under Example 2 for reaction with 2-hydroxyethylamine to give 3-benzyloxy-1-(3'-hydroxypropyl)-2-methylpyrid-4-one. This is deprotected using the procedure described under Example 2 to give 3-hydroxy-1-(3'-hydroxypropyl)-2-methylpyrid-4-one as white crystals, m.p. 111-113°C; $\nu_{max}$ (nujol) 1630, 3150, 3350 $cm^{-1}$; $\delta(d_6DMSO)$, 1.8 (m, 2H), 2.4 (s, 3H), 3.35 (t, 2H), 4.33 (t, 2H), 7.3 (d, 1H), 8.2 (d, 1H).

Example 4

The preparation of 3-hydroxy-1-(4'-hydroxybutyl)-2-methylpyrid-4-one

3-Benzyloxy-2-methyl-4-pyrone, prepared as described under Example 1, is reacted with 1-amino-4-hydroxybutane under substantially similar conditions to those described under Example 2 for reaction with 2-hydroxyethylamine to give 3-benzyloxy-1-(4'-hydroxybutyl)-2-methylpyrid-4-one. This is deprotected using the procedure described under Example 2 to give 3-hydroxy-1-(4'-hydroxybutyl)-2-methylpyrid-4-one as white crystals, m.p. 126-128°C; $\nu_{max}$ (nujol) 1630, 3350 $cm^{-1}$; $\delta(d_6DMSO)$, 1.5 (m, 4H), 2.45 (s, 3H), 3.35 (t, 2H), 4.30 (t, 2H), 7.25 (d, 1H), 8.2 (d, 1H).

Example 5

The preparation of 3-hydroxy-1-(5'-hydroxypentyl)-2-methylpyrid-4-one

3-Benzyloxy-2-methyl-4-pyrone, prepared as described under Example 1, is reacted with 1-amino-5-hydroxypentane under substantially similar conditions to those described under Example 2 for reaction with 2-hydroxyethylamine to give 3-benzyloxy-1-(5'-hydroxypentyl)-2-methylpyrid-4-one. This is deprotected using the procedure described under Example 2 to give 3-hydroxy-1-(5'-hydroxypentyl)-2-methylpyrid-4-one as white crystals, m.p. 136-138°C; $\nu_{max}$ (nujol) 1625, 3350 $cm^{-1}$; $\delta(d_6DMSO)$, 1.3 (m, 6H), 2.40 (s, 3H), 3.25 (t, 2H), 4.20 (t, 2H), 7.20 (d, 1H), 8.12 (d, 1H).

Example 6

The preparation of 1-(5'-acetoxypentyl)-3-hydroxy-2-methylpyrid- 4-one

3-Hydroxy-1-(5'-hydroxypentyl)-2-methylpyrid-4-one, prepared as described under Example 5, (2 g) is dissolved in glacial acetic acid containing about 1% w/v of hydrogen bromide and the solution is refluxed for 2 hours. The resultant mixture is subjected to rotary evaporation and the residue crystallised from aqueous ethanol to give 1-(5'-acetoxypentyl)-3-hydroxy-2-methylpyrid-4-one in 65% yield, m.p. 132-133°C; $\nu_{max}$ (nujol) 1520, 1540, 1580, 1635, 1735 $cm^{-1}$; $\delta(d_6DMSO)$, 1.6 (m, 6H), 2.1 (s, 3H), 2.4 (s, 3H), 4.05 (m, 4H), 6.4 (d, 1H), 7.3 (d, 1H).

Example 7

The preparation of 1-(2'-ethoxycarbonylethyl)-3-hydroxy-2-methylpyrid-4-one 3-Benzyloxy-1-(2'-carboxyethyl)-2-methylpyrid-4-one

3-Benzyloxy-2-methyl-4-pyrone, prepared as described under Example 1, (20 g) and beta-alanine (9 g) are dissolved in 3:2 v/v water/ethanol (500 ml) and containing NaOH (10 g) to provide a solution with a pH of at least 13. The solution is refluxed for 15 minutes whereupon it turns from a light orange to an intense red colour. The solution is acidified to pH 7.0 and the ethanol is removed by rotary evaporation. The resulting aqueous solution is washed twice with ethylacetate (100 ml). This solution is then subjected to rotary evaporation until the volume is reduced to 100 ml and this reduced volume of solution is acidified to pH 3.0 to give a white precipitate, which is filtered off to give 3-benzyloxy-1-(2'-carboxyethyl)-2-methylpyrid-4-one in 70% yield, m.p. 156-157°C.

1-(2'-Carboxyethyl)-3-hydroxy-2-methylpyrid-4-one

3-Benzyloxy-1-(2'-carboxyethyl)-2-methylpyrid-4-one is subjected to hydrogenation in aqueous ethanol (1:1 v/v) in the presence of platinum/carbon catalyst (100 mg per gram of pyridone) for 8 hours at 20°C and atmospheric pressure. Filtration, followed by rotary evaporation of the filtrate, yields a white solid which on recrystallisation from acetone and

diethylether gives 1-(2'-carboxyethyl)-3-hydroxy-2-methylpyrid-4-one hydrochloride in 55% yield, $\nu_{max}$ (nujol) 1590, 1620, 1720 $cm^{-1}$; $\delta(d_6DMSO)$, 2.5 (s 3H), 2.8 (t, 2H), 4.45 (t, 2H), 7.35 (d, 1H), 8.2 (d, 1H).

1-(2'-Ethoxycarbonylethyl)-3-hydroxy-2-methylpyrid-4-one

1-(2'-Carboxyethyl)-3-hydroxy-2-methylpyrid-4-one hydrochloride (2 g) is dissolved in ethanol saturated with hydrogen chloride and the solution is refluxed for 3 hours. Rotary evaporation of the solution yields a white solid which on recrystallisation from ethanol gives 1-(2'-ethoxycarbonylethyl)-3-hydroxy-2-methylpyrid-4-one hydrochloride in 75% yield, m.p. 127-130°C; $\nu_{max}$ (nujol) 1530, 1620, 1720 $cm^{-1}$; $\delta(d_6DMSO)$, 1.1 (t 3H), 2.5 (s, 3H), 2.9 (t, 2H), 4.0 (q, 2H), 4.5 (t, 2H), 7.4 (d, 1H), 8.2 (d, 1H).

Example 8

The preparation of 3-hydroxy-1-(2'-methoxycarbonylethyl)-2-methylpyrid-4-one

1-(2'-Carboxyethyl)-3-hydroxy-2-methylpyrid-4-one hydrochloride (2 g), prepared as described under Example 7, is dissolved in methanol saturated with hydrogen chloride and the solution is refluxed for 3 hours. Rotary evaporation of the solution yields a white solid which on recrystallisation from methanol gives 3-hydroxy-1-(2'-methoxycarbonylethyl)-2-methylpyrid-4-one hydrochloride in 80% yield, m.p. 140-141°C; $\nu_{max}$ 1545, 1595, 1645, 1730, 1750 $cm^{-1}$; $\delta(d_6DMSO)$, 2.45 (s 3H), 2.85 (t, 2H), 3.5 (s, 3H), 4.45 (t, 2H), 7.30 (d, 1H), 8.15 (d, 1H).

Example 9

The preparation of 3-hydroxy-1-[2'-(N-propylcarbamoyl)-ethyl]-2-methylpyrid-4-one 3-Benzyloxy-1-[2'-(succinimido-oxycarbonyl)-ethyl]-2-methylpyrid-4-one

3-Benzyloxy-1-(2'-carboxyethyl)-2-methylpyrid-4-one, prepared as described under Example 7, (2.2 g) is dissolved in DMF (25 ml) and to the solution is added N-hydroxysuccinimide (1 g). This solution is cooled and to it is added dicyclohexylcarbodiimide (1.8 g) in DMF (5 ml). The mixture is allowed to stand overnight giving a dark coloured solution and white precipitates. The precipitate is removed by filtration and washed with DMF (3 ml). The solution is rotary evaporated to dryness and triturated with diethylether to give 3-benzyloxy-1-[2'-succinimido-oxycarbonyl)-ethyl]-2-methylpyrid-4-one hydrochloride as a white solid in 55% yield m.p. 45-47°C.

3-Benzyloxy-1-[2'-(N-propylcarbamoyl)-ethyl]-2-methylpyrid- 4-one

3-Benzyloxy-1-[2'-(succinimido-oxycarbonyl)-ethyl]-2- methylpyrid-4-one (1 g) is dissolved in chloroform (100 ml) and to this solution is added propylamine in a 2 molar excess. The mixture is allowed to stand for 15 minutes and the N-hydroxysuccinimide liberated by the reaction is then extracted with 1M $NaHCO_3$ solution (2 x 25 ml). The organic layer is dried over $Na_2SO_4$, filtered and evaporated to dryness to give 3-benzyloxy-1-[2'-(N-propylcarbamoyl)-ethyl]-2-methylpyrid-4-one as a white solid in 40% yield, m.p. 146-147°C.

3-Hydroxy-1-[2'-(N-propylcarbamoyl)-ethyl]-2-methylpyrid-4-one

3-Benzyloxy-1-[2'-N-propylcarbamoyl)-ethyl]-2-methyl- pyrid-4-one (5 g) is dissolved in 50:50 v/v aqueous ethanol. Platinum/carbon catalyst (100 mg) is added and the solution is hydrogenated at 20°C and atmospheric pressure for 8 hours. The mixture is filtered, the filtrate subjected to rotary evaporation and the resulting residue recrystallised from ethanol to give 3-hydroxy-1-[2'-(N-propylcarbamoyl)-ethyl]-pyrid-4-one, m.p. 113-114°C, $\nu_{max}$ (nujol) 1505, 1550, 1570, 1630, 1705, 3080, 3200 $cm^{-1}$; $\delta(d_6DMSO)$ 0.6 (t, 3H), 1.2 (sextuplet, 2H), 2.5 (s, 3H), 2.6, 2.8 (overlapping t and q, 4H), 4.5 (t, 2H), 7.2 (d, 1H), 8.1 (overlapping d and t, 2H).

Example 10

The preparation of 3-hydroxy-6-hydroxymethyl-1-methylpyrid-4-one 3-Benzyloxy-6-benzyloxymethyl-4-pyrone

Kojic acid (3-hydroxy-6-hydroxymethyl-4-pyrone( (11.5 g) in methanol (90 ml) is added to an aqueous solution of sodium hydroxide (3 g in 10 ml). Benzylchloride (10.2 g) is added and the mixture is stirred and refluxed for 6 hours. On cooling, crystals are obtained which are recrystallised from methanol to give 3-benzyloxy-6-benzyloxymethyl-4-pyrone as white crystals (10 g), m.p. 103-131°C; $\delta(CDCl_3)$ 4.25 (s, 2H), 4.99 (s, 2H), 6.25 (s, 1H), 7.3 (s, 5H), 8.1 (s, 1H).

3-Hydroxy-6-hydroxymethyl-1-methylpyrid-4-one

3-Benzyloxy-6-benzyloxy-4-pyrone (5.0 g) and methylamine hydrochloride (1.56 g) are dissolved in aqueous ethanbol (300 ml of 2:1 $H_2O$-$C_2H_5OH$ by volume containing 2 g of sodium hydroxide). The reaction mixture is stirred at room temperature for 6 days and then acidified with concentrated HCl to pH 2.0. The yellow mixture is evaporated to dryness and the resulting solid residue is heated under reflux with concentrated HCl (50 ml) for 30 minutes. The product is rotary evaporated to dryness yielding a brown residue which on trituration with acetone forms crystals. Recrystallisation of these from ethanol gives 3-hydroxy-6-hydroxymethyl-1-methylpyrid-4-one in 15% yield, m.p. 185-186°C; δ($d_6$DMSO), 3.9 (s, 3H), 4.6 (s, 2H), 7.4 (s, 1H), 8.3 (s, 1H).

Example 11

The preparation of 1-ethyl-3-hydroxy-6-hydroxymethylpyrid-4-one

3-Benzyloxy-6-benzyloxymethyl-4-pyrone, prepared as described under Example 10, is reacted with ethylamine hydrochloride under substantially similar conditions to those described under Example 10 for reaction with methylamine hydrochloride to give 1-ethyl-3-hydroxy-6-hydroxymethylpyrid-4-one as white crystals, m.p. 143-145°C; δ($d_6$DMSO) 1.1 (t, 3H), 4.0 (q, 2H), 4.3 (s, 2H), 7.2 (s, 1H), 8.1 (s, 1H).

Example 12

Partition data on 3-hydroxypyrid-2- and -4-ones and their iron complexes

The partition coefficient $K_{part}$ being the ratio (concentration of compound in n-octanol)/)concentration of compound in aqueous phase) on partition between n-octanol and aqueous tris hydrochloride (20 mM, pH 7.4), is measured by spectrophotometry at 20°C for various of the compounds of the previous Examples and for their 3:1 iron(III) complexes at ($10^{-4}$M by spectrophotometry. The solutions of the complexes are either produced by dissolving the pre-formed complex in the aqueous tris hydrochloride or are prepared in situ in the buffer by the admixture of a 3:1 molar ratio of the pyridone and ferric chloride, the pH thereafter being readjusted to 7.4 if necessary (the same product will be obtained in the buffer solution irrespective of whether the pyridone used is in a salt form or not). Acid washed glassware is used throughout and, following mixing of 5 ml of the $10^{-4}$M aqueous solution with 5 ml n-octanol for 1 minute, the aqueous n-octanol mixture is centrifuged at 1,000 g for 30 seconds. The two resulting phases are separated for a concentration determination by spectrophotometry on each. For the free hydroxypyridones, the range 220-240 nm is used for concentration determinations whilst for the iron complexes, the range 340-360 nm is used.

Values typical of those obtained are shown in Table 1.

Table 1

| Partition coefficients | | |
|---|---|---|
| Compound | Partition coefficient $K_{part}$ | |
| | Free compound | Iron complex [$Fe^{III}$-(compound)$_3$] |
| 3-hydroxy-1-(2'-hydroxyethyl)-2-methylpyrid-4-one | <0.002 | <0.002 |
| 3-hydroxy-1-(3'-hydroxypropyl)-2-methylpyrid-4-one | 0.10 | 0.06 |
| 3-hydroxy-1-(4'-hydroxybutyl)-2-methylpyrid-4-one | 0.14 | 0.03 |
| 3-hydroxy-1-(5'-hydroxypentyl)-2-methylpyrid-4-one | 0.28 | 0.04 |
| 1-(5'-acetoxypentyl-3-hydroxy-2-methylpyrid-4-one | 0.45 | 0.03 |
| 3-hydroxy-6-hydroxymethyl-1-methyylpyrid-4-one | 0.03 | 0.01 |
| 1-ethyl-3-hydroxy-6-hydroxy-methylpyrid-4-one | 0.06 | 0.07 |

Example 13

In vitro tests of iron binding capacity

The 3-hydroxypyrid-4-ones used in this Example were prepared as described in various of the previous Examples.

(1) Mobilisation of iron from ferritin

Horse spleen ferritin (Sigma) was used without further purification and its iron content was estimated spectrophotometrically at 420 nm. The ferritin solution in phosphate buffered saline (Dulbecco-OXOID, $10^{-6}$ M, pH 7.4) was enclosed in a Visking dialysis tube and dialysed against a 3 x $10^{-3}$ M buffered solution of one of various pyridones as indicated in Table 2. The absorption spectrum of the resulting iron (III) complex in the dialysis solution was recorded after 6 and 24 hours. For comparative purposes, the procedure was repeated using a blank control.

The results are shown in Table 2 where the percentage of ferritin-bound iron removed by the compound under test is shown. For comparative purposes, results reported in the literature for similar tests with 1 x $10^{-3}$ M desferrioxamine (Crichton et al., J. Inorganic Biochem., 1980, 13, 305) and with 6 x $10^{-3}$ M LICAMS (Tufano et al., Biochem. Biophys. Acta, 1981, 668, 420) are also given in the Table. It will be seen that the pyridone compounds are able to remove iron effectively from ferritin in contrast with desferrioxamine and LICAMS (although the latter will remove iron in the presence of ascorbic acid such a mixture is very difficult to manage clinically). These results shown in Table 2 have been confirmed by separating apoferritin (in admixture with ferritin) and the particular hydroxypyridone iron (III) complex from the reaction product in each case by chromatography on Sephadex G10.

Table 2

| Removal of iron from ferritin | | |
|---|---|---|
| Compound | Percentage of iron removed | |
| | 6 hours | 24 hours |
| Control | 0 | 0 |
| 3-hydroxy-1-(2'-hydroxyethyl)-2-methylpyrid-4-one | 31 | 49 |
| 3-hydroxy-1-(2'-methoxycarbonylethyl)-2-methylpyrid-4-one | 22 | 45 |
| 3-hydroxy-6-hydroxymethyl-1-methylpyrid-4-one | 25 | 49 |
| Desferrioxamine (1 mM) | 1.5 | - |
| LICAMS (6 mM) | 0 | - |
| LICAMS (6 mM + 12 mM ascorbic acid) | 7 | - |

(2) Mobilisation of iron from transferrin

Human transferrin (Sigma) was loaded with iron (III) by the method of Bates and Schlaback. J. Biol. Chem. (1973) 248, 3228. $^{59}$Iron (III) transferrin ($10^{-5}$ M) was incubated with a 4 x $10^{-3}$ M solution in tris HCl (0.1 M, pH 7.4) of one of various pyridones as indicated in Table 3 for periods of 4 hours and 18 hours. The solution was then dialysed against phosphate buffered saline for 24 hours. The $^{59}$Fe remaining in the dialysis tube was then recorded. For comparative purposes, this procedure was repeated with desferrioxamine using incubation for both 4 hours and 18 hours and with EDTA using incubation for 4 hours only.

The results are shown in Table 3 in terms of the percentage of transferrin bound iron removed by the compound under test. It will be seen that the pyrid-4-one compounds are very effective at iron removal, as compared with desferrioxamine or EDTA, after only 4 hours.

The results shown in Table 3 have been confirmed by separating apotransferrin (in admixture with transferrin) and the particular hydroxypyridone iron complex from the reaction product in each case by chromatography on Sephadex G10.

Table 3

| Removal of iron from transferrin | | |
|---|---|---|
| Compound | Percentage of iron removed | |
| | 4 hours | 18 hours |
| 3-hydroxy-1-(2'-hydroxyethyl)-2-methylpyrid-4-one | 94 | 96 |
| 3-hydroxy-6-hydroxymethyl-1-methylpyrid-4-one | 93 | 97 |
| Desferrioxamine | 17 | 22 |
| EDTA | 27 | 67 |

Example 14

In vivo tests of iron binding capacity

The 3-hydroxypyrid-4-ones used in this Example were prepared as described in various of the previous Examples.

Mice were injected intraperitoneally with iron dextran (2 mg) at weekly intervals over a four week period. Two weeks after the final injection, the mice were injected via the tail vein with $^{59}Fe$ lactoferrin (human lactoferrin, 1 mg per injection 2 μCi). The mice were then caged individually. After a ten day period, 1-acetyl-3-hydroxypyrid-2-one was administered to groups of 8 mice at 10 mg per mouse either intraperitoneally or intragastrically (in each case 3 of the mice received only one dose whilst 5 received 2 doses at a 24 hour interval). The excretion of iron was recorded at either 12 or 24 hourly intervals over a three day period before and a two day period after administration of the compound. For comparative purposes, the procedure was repeated with a blank control and with desferrioxamine, also at 10 mg per mouse (the intraperitoneally treated mice receiving one dose of desferrioxamine and the intragastrically treated mice two doses at a 24 hour interval).

The results are shown in Table 4, being given on the basis of the control representing 100% excretion, and illustrate the particular advantage of the pyridones as compared with desferrioxamine for oral administration the levels of iron excreted on intragastric administration being higher for all the pyridones tested than for desferroxamine. It should be mentioned that the large standard deviation (SD) values are somewhat misleading as uniformly positive results can yield high SDs which might be taken to suggest that the results are not significantly different from zero. However, this is not the case here, the large SD values being a consequence of the large range among the positive responses.

Table 4

| Excretion of iron in vivo | | | | |
|---|---|---|---|---|
| | Intraperitoneal Administration | | Intragastric Administration | |
| | Number of Mice | Excretion of $^{59}Fe\pm SD$ percent | Number of Mice | Excretion of $^{59}Fe\pm SD$ percent |
| Control | 12 | 100 ± 10 | - | - |
| 3-hydroxy-1-(2'-hydroxyethyl)-2-methylpyrid-4-one | 11 | 170 ± 49 | - | - |
| 3-hydroxy-1-(5'-hydroxypentyl)-2-methylpyrid-4-one | 9 | 149 ± 59 | 9 | 111 ± 22 |
| Desferrioxamine | 5 | 414 ± 100 | 4 | 88 ± 17 |

## Claims

1. A compound of the formula (1)

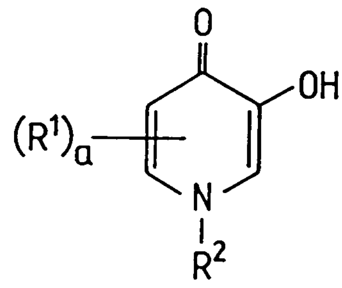

(1)

in which each $R^1$ separately is a $C_{1-6}$ aliphatic hydrocarbon coup or a $C_{1-8}$ aliphatic hydrocarbon group substituted by a formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen or hydroxy group,
$R^2$ is a $C_{1-6}$ aliphatic hydrocarbon group, a formyl or ($C_{1-4}$ alkyl)-carbonyl group, or a $C_{1-8}$ aliphatic hydrocarbon group substituted by one or more substituents selected from formyl, ($C_{1-4}$ alkyl)-carbonyl, carbamoyl, sulphamoyl, mono- and di-$C_{1-6}$ aliphatic hydrocarbyl N-substituted carbamoyl and N-substituted sulphamoyl, formylamino, ($C_{1-6}$ aliphatic hydrocarbyl)-carbonylamino, ($C_{1-6}$ aliphatic hydrocarbyl)-sulphonylamino, mono-$C_{1-6}$ aliphatic hydrocarbyl N-substituted formylamino, N-substituted ($C_{1-6}$ aliphatic hydrocarbyl)-carbonylamino and N-substituted ($C_{1-6}$ aliphatic hydrocarbyl)-sulphonylamino, formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen ad hydroxy groups, and a is 0, 1, 2 or 3,
but excluding compounds in which each $R^1$ and $R^2$ are selected from $C_{1-6}$ aliphatic hydrocarbon groups and those in which the grouping >N-$R^2$ contains either a group

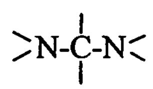

or a group

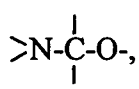

or a physiologically acceptable salt thereof, for use in therapy.

**2.** A compound of the formula (1)

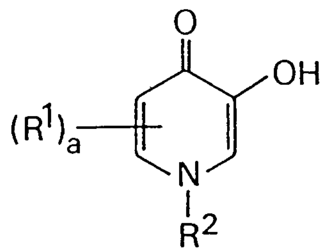

(1)

in which each $R^1$ separately is a $C_{1-6}$ aliphatic hydrocarbon group or a $C_{1-8}$ aliphatic hydrocarbon group substituted by a formyloxy, ($C_{1-6}$ aliphatic hydrocarbyl)-carbonyloxy, ($C_{1-6}$ aliphatic hydrocarbyl)-oxycarbonyl, ($C_{1-6}$ aliphatic hydrocarbyl)-sulphonyloxy, ($C_{1-6}$ aliphatic hydrocarbyl)-oxysulphonyl, halogen or hydroxy group,
$R^2$ is a $C_{1-6}$ aliphatic hydrocarbon group, a formyl or ($C_{1-4}$ alkyl)- carbonyl group, or a $C_{1-8}$ aliphatic hydrocarbon group substituted by one or more substituents selected from formyl, ($C_{1-4}$ alkyl)-carbonyl, carbamoyl, sulphamoyl, mono- and di-$C_{1-6}$ aliphatic hydrocarbyl N-substituted carbamoyl and N-substituted sulphamoyl, formylamino, ($C_{1-6}$ aliphatic hydrocarbyl)-carbonylamino, ($C_{1-6}$ aliphatic hydrocarbyl)- sulphonylamino, mono-$C_{1-6}$ aliphatic hydrocarbyl N-substituted formylamino, N-substituted ($C_{1-6}$ aliphatic hydrocarbyl)-carbonylamino and N-substituted ($C_{1-6}$ aliphatic hydrocarbyl)-sulphonylamino, formyloxy, ($C_{1-6}$ aliphatic hydrocarbyl)- carbonyloxy, ($C_{1-6}$ aliphatic hydrocarbyl)-oxycarbonyl, ($C_{1-6}$ aliphatic hydrocarbyl)- sulphonyloxy, ($C_{1-6}$ aliphatic hydrocarbyl)-oxysulphonyl, halogen and hydroxy groups, and a is 1, 2 or 3;
but excluding compounds in which each $R^1$ and $R^2$ is selected from $C_{1-6}$ aliphatic hydrocarbon groups, compounds in which the grouping >N-$R^2$ contains either a group

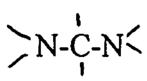

or a group

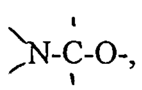

and compounds in which $(R^1)_a$ is a 6-chloromethyl group and $R^2$ is a methyl group, $(R^1)_a$ is a 6-hydroxymethyl group and $R^2$ is a methyl or ethyl group, or $(R^1)_a$ is a 2-methyl group and $R^2$ is a 2-hydroxyethyl or methoxycarbonylmethyl group; or a physiologically acceptable salt thereof.

**3.** A compound according to Claim 1 or 2, in which a is 0, 1 or 2.

**4.** A compound according to Claim 3, in which a is 1 or 2.

**5.** A compound according to any of the preceding claims, which has a group $R^1$ at the 2-position.

**6.** A compound according to any of the preceding claims, in which $R^1$ is a $C_{1-4}$ aliphatic hydrocarbon group or a $C_{1-3}$ alkyl group substituted by a hydroxy group.

**7.** A compound according to Claim 4, in which a is 1 and $R^1$ is a $C_{1-6}$ alkyl group at the 2-position or at the 6-position or a is 2 and $R^1$ is a $C_{1-6}$ alkyl group at the 2- and 6-positions.

**8.** A compound according to Claim 6 or 7, in which $R^1$ is methyl, ethyl, propyl or isopropyl.

9. A compound according to Claim 4, in which a is 1 and $R^1$ is a methyl or ethyl group at the 2- position or a methyl group at the 6-position or a is 2 and $R^1$ is a methyl group at each of the 2- and 6-positions.

10. A compound according to any of the preceding claims in which $R^2$ is a substituted $C_{1-6}$ aliphatic hydrocarbon group.

11. A compound according to any of the preceding claims, in which any hydrocarbyl group in the substituent of a substituted aliphatic hydrocarbon group $R^2$ is a $C_{1-4}$ group.

12. A compound according to any of the preceding claims, in which $R^2$ is a group $-(CH_2)_mOH$ in which m is an integer from 2 to 4.

13. A compound according to any of Claims 1 to 11, in which $R^2$ is a $C_{1-6}$ aliphatic hydrocarbon group substituted by a formyloxy, ($C_{1-4}$ alkyl)-carbonyloxy, ($C_{1-4}$ alkyl)-oxycarbonyl, ($C_{1-4}$ alkyl)-sulphonyloxy or ($C_{1-4}$ alkyl)-oxysulphonyl group.

14. A compound according to any of Claims 1 to 11, in which $R^2$ is a $C_{1-6}$ aliphatic hydrocarbon group substituted by a carbamoyl, sulphamoyl, mono- or di-$C_{1-4}$ alkyl N-substituted carbamoyl or N-substituted sulphamoyl, formylamino ($C_{1-4}$ alkyl)-carbonylamino, ($C_{1-4}$ alkyl)-sulphonylamino, mono-$C_{1-4}$ alkyl N-substituted formylamino, N-substituted ($C_{1-4}$ alkyl)-carbonylamino or N-substituted ($C_{1-4}$ alkyl)-sulphonylamino group.

15. A compound according to any of the preceding claims in which $R^2$ is a substituted $C_{1-4}$ alkyl group.

16. A compound according to any of Claims 1 to 9, in which $R^2$ is a $C_{1-4}$ alkyl group.

17. A compound according to Claim 1, being 3-hydroxy-1-(2'-hydroxyethyl)-2-methylpyrid-4-one or a physiologically acceptable salt thereof.

18. A compound according to Claim 1 or 2, being 3-hydroxy-1-(3'-hydroxypropyl)-2-methypyrid-4-one or a physiologically acceptable salt thereof.

19. A pharmaceutical composition comprising a compound according to Claim 2, together with a physiologically acceptable diluent or carrier.

20. A pharmaceutical composition according to Claim 19, in which the diluent or carrier is a physiologically acceptable solid carrier.

21. A pharmaceutical composition according to Claim 19, in which the diluent or carrier is a physiologically acceptable liquid.

22. A pharmaceutical composition according to Claim 19, 20 or 21, which is adapted for oral administration.

23. A pharmaceutical composition according to Claim 19, 20 or 21, which is in injectable form or in suppository form.

24. A pharmaceutical composition comprising a compound of formula (1) as defined in Claim 1, together with a physiologically acceptable diluent or carrier which is a solid composition adapted for oral administration or a liquid injectable composition that is sterile and pyrogen free.

25. A pharmaceutical composition according to any of Claims 19 to 24, in unit dosage form.

26. A pharmaceutical composition according to any of Claims 19 to 25, in which the compound of formula (1) is as defined in any of Claims 3 to 18.

27. The use of a compound of formula (1) as defined in Claim 1, for the manufacture of a medicament for use in effecting a reduction in toxic levels of iron in a patient's body.

28. The use according to Claim 27, in which the compound of formula (1) is as defined in any of Claims 3 to 18.

## Patentansprüche

1. Verbindung der Formel (1)

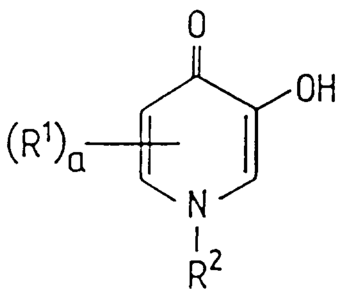

(1)

in der jedes $R^1$ getrennt eine aliphatische $C_1$-$C_6$-Kohlenwasserstoffgruppe oder eine aliphatische $C_1$-$C_8$-Kohlenwasserstoffgruppe, substituiert durch eine Formyloxy, (aliphatische $C_1$-$C_6$-Kohlenwasserstoff)-carbonyloxy, (aliphatische $C_1$-$C_6$-Kohlenwasserstoff)-oxycarbonyl, (aliphatische $C_1$-$C_6$-Kohlenwasserstoff)-sulfonyloxy, (aliphatische $C_1$-$C_6$-Kohlenwasserstoff)-oxysulfonylgruppe, Halogen oder Hydroxygruppe bedeutet,
$R^2$ eine aliphatische $C_1$-$C_6$-Kohlenwasserstoffgruppe, eine Formyl- oder ($C_1$-$C_4$-Alkyl)-carbonylgruppe oder eine aliphatische $C_1$-$C_8$-Kohlenwasserstoffgruppe, substituiert durch einen oder mehrere Substituenten, ausgewählt aus Formyl-, ($C_1$-$C_4$-Alkyl)-carbonyl-, Carbamoyl-, Sulfamoyl-, aliphatischen Mono- und Di-$C_1$-$C_6$-Kohlenwasserstoff-N-substituierten Carbamoyl- und N-substituierten Sulfamoyl-, Formylamino-, (aliphatischen $C_1$-$C_6$-Kohlenwasserstoff)-carbonylamino, (aliphatischen $C_1$-$C_6$-Kohlenwasserstoff)-sulfonylamino, aliphatischen Mono-$C_1$-$C_6$-Kohlenwasserstoff-N-substituierten Formylamino-, N-substituierten (aliphatischen $C_1$-$C_6$-Kohlenwasserstoff)-carbonylamino- und N-substituierten (aliphatischen $C_1$-$C_6$-Kohlenwasserstoff)-sulfonylamino-, Formyloxy-, (aliphatischen $C_1$-$C_6$-Kohlenwasserstoff)-carbonyloxy, (aliphatischen $C_1$-$C_6$-Kohlenwasserstoff)-oxycarbonyl, (aliphatischen $C_1$-$C_6$-Kohlenwasserstoff)-sulfonyloxy, (aliphatischen $C_1$-$C_6$-Kohlenwasserstoff)-oxysulfonylgruppen, Halogen und Hydroxygruppe bedeutet und a 0, 1, 2 oder 3 ist,
aber mit Ausnahme von Verbindungen, bei denen jedes $R^1$ und $R^2$ ausgewählt ist aus aliphatischen $C_1$-$C_6$-Kohlenwasserstoffgruppen und solchen, bei denen die Gruppierung $>N-R^2$ entweder eine Gruppe

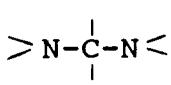

oder eine Gruppe

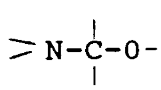

enthält,
oder ein physiologisch annehmbares Salz davon zur Verwendung bei der Therapie.

**2.** Verbindung der Formel (1)

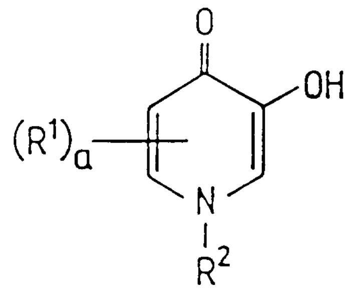

(1)

in der jedes $R^1$ getrennt eine aliphatische $C_1$-$C_6$-Kohlenwasserstoffgruppe oder eine aliphatische $C_1$-$C_8$-Kohlenwasserstoffgruppe, substituiert durch eine Formyloxy, (aliphatische $C_1$-$C_6$-Kohlenwasserstoff)-carbonyloxy, (aliphatische $C_1$-$C_6$-Kohlenwasserstoff)-oxycarbonyl, (aliphatische $C_1$-$C_6$-Kohlenwasserstoff)-sulfonyloxy, (aliphatische $C_1$-$C_6$-Kohlenwasserstoff)-oxysulfonylgruppe, Halogen oder Hydroxygruppe bedeutet,
$R^2$ eine aliphatische $C_1$-$C_6$-Kohlenwasserstoffgruppe, eine Formyl- oder ($C_1$-$C_4$-Alkyl)-carbonylgruppe oder eine aliphatische $C_1$-$C_8$-Kohlenwasserstoffgruppe, substituiert durch einen oder mehrere Substituenten, ausgewählt aus Formyl-, ($C_1$-$C_4$-Alkyl)-carbonyl-, Carbamoyl-, Sulfamoyl-, aliphatischen Mono- und Di-$C_1$-$C_6$-Kohlenwasserstoff-N-substituierten Carbamoyl- und N-substituierten Sulfamoyl-, Formylamino-, (aliphatischen $C_1$-$C_6$-Kohlenwasserstoff)-carbonylamino, (aliphatischen $C_1$-$C_6$-Kohlenwasserstoff)-sulfonylamino, aliphatischen Mono-$C_1$-$C_6$-Kohlenwasserstoff-N-substituierten Formylamino-, N-substituierten (aliphatischen $C_1$-$C_6$-Kohlenwasserstoff)-carbonylamino- und N-substituierten (aliphatischen $C_1$-$C_6$-Kohlenwasserstoff)-sulfonylamino-, Formyloxy-, (aliphatischen $C_1$-$C_6$-Kohlenwasserstoff)-carbonyloxy, (aliphatischen $C_1$-$C_6$-Kohlenwasserstoff)-oxycarbonyl, (aliphatischen $C_1$-$C_6$-Kohlenwasserstoff)-sulfonyloxy, (aliphatischen $C_1$-$C_6$-Kohlenwasserstoff)-oxysulfonylgruppen, Halogen und Hydroxygruppe bedeutet und a 1, 2 oder 3 ist,
aber ausschließlich Verbindungen, bei denen jedes $R^1$ und $R^2$ ausgewählt ist aus aliphatischen $C_1$-$C_6$-Kohlenwasserstoffgruppen, Verbindungen, bei denen die Gruppierung >N-$R^2$ entweder eine Gruppe

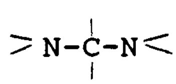

oder eine Gruppe

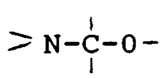

enthält, und Verbindungen, bei denen $(R^1)_a$ eine 6-Chlormethylgruppe und $R^2$ eine Methylgruppe ist, $(R^1)_a$ eine 6-Hydroxymethylgruppe und $R^2$ eine Methyl- oder Ethylgruppe ist, oder $(R^1)_a$ eine 2-Methylgruppe und $R^2$ eine 2-Hydroxyethyl- oder Methoxycarbonylmethylgruppe ist, oder ein physiologisch annehmbares Salz davon.

**3.** Verbindung nach Anspruch 1 oder 2, wobei a 0, 1 oder 2 ist.

**4.** Verbindung nach Anspruch 3, wobei a 1 oder 2 ist.

**5.** Verbindung nach einem der vorangehenden Ansprüche, die eine Gruppe $R^1$ in 2-Stellung enthält.

**6.** Verbindung nach einem der vorangehenden Ansprüche, wobei $R^1$ eine aliphatische $C_1$-$C_4$-Kohlenwasserstoffgruppe oder eine $C_1$-$C_3$-Alkylgruppe, substituiert durch eine Hydroxygruppe, ist.

**7.** Verbindung nach Anspruch 4, wobei a 1 ist und $R^1$ eine $C_1$-$C_6$-Alkylgruppe in 2-Stellung oder in 6-Stellung ist oder a 2 ist und $R^1$ eine $C_1$-$C_6$-Alkylgruppe in 2- und 6-Stellung ist.

**8.** Verbindung nach Anspruch 6 oder 7, wobei $R^1$ Methyl, Ethyl, Propyl oder Isopropyl ist.

9. Verbindung nach Anspruch 4, wobei a 1 ist und $R^1$ eine Methyl- oder Ethylgruppe in 2-Stellung oder eine Methylgruppe in 6-Stellung ist oder a 2 ist und $R^1$ eine Methylgruppe jeweils in 2- und 6-Stellung ist.

10. Verbindung nach einem der vorangehenden Ansprüche, wobei $R^2$ eine substituierte aliphatische $C_1$-$C_6$-Kohlenwasserstoffgruppe ist.

11. Verbindung nach einem der vorangehenden Ansprüche, wobei jede Kohlenwasserstoffgruppe in dem Substituenten einer substituierten aliphatischen Kohlenwasserstoffgruppe $R^2$ eine $C_1$-$C_4$-Gruppe ist.

12. Verbindung nach einem der vorangehenden Ansprüche, wobei $R^2$ eine Gruppe $-(CH_2)_mOH$ ist, in der m eine ganze Zahl von 2 bis 4 bedeutet.

13. Verbindung nach einem der Ansprüche 1 bis 11, wobei $R^2$ eine aliphatische $C_1$-$C_6$-Kohlenwasserstoffgruppe, substituiert durch eine Formyloxy-, ($C_1$-$C_4$-Alkyl)-carbonyloxy-, ($C_1$-$C_4$-Alkyl)-oxycarbonyl-, ($C_1$-$C_4$-Alkyl)-sulfonyloxy- oder ($C_1$-$C_4$-Alkyl)-oxysulfonylgruppe ist.

14. Verbindung nach einem der Ansprüche 1 bis 11, wobei $R^2$ eine aliphatische $C_1$-$C_6$-Kohlenwasserstoffgruppe, substituiert durch eine Carbamoyl-, Sulfamoyl-, Mono- oder Di-$C_1$-$C_4$-alkyl)-N-substituierte Carbamoyl- oder N-substituierte Sulfamoyl-, Formylamino-($C_1$-$C_4$-alkyl)-carbonylamino-, ($C_1$-$C_4$-Alkyl)-sulfonylamino-, Mono-$C_1$-$C_4$-alkyl-N-substituierte Formylamino-, N-substituierte ($C_1$-$C_4$-Alkyl)-carbonylamino- oder N-substituierte ($C_1$-$C_4$-Alkyl)-sulfonylaminogruppe ist.

15. Verbindung nach einem der vorangehenden Ansprüche, wobei $R^2$ eine substituierte $C_1$-$C_4$-Alkylgruppe ist.

16. Verbindung nach einem der Ansprüche 1 bis 9, wobei $R^2$ eine $C_1$-$C_4$-Alkylgruppe ist.

17. Verbindung nach Anspruch 1, die 3-Hydroxy-1-(2'-hydroxyethyl)-2-methylpyrid-4-on oder ein physiologisch annehmbares Salz davon ist.

18. Verbindung nach Anspruch 1 oder 2, die 3-Hydroxy-1-(3'-hydroxypropyl)-2-methypyrid-4-on oder ein physiologisch annehmbares Salz davon ist.

19. Pharmazeutisches Mittel, umfassend eine Verbindung nach Anspruch 2 zusammen mit einem physiologisch annehmbaren Verdünnungsmittel oder Träger.

20. Pharmazeutisches Mittel nach Anspruch 19, wobei das Verdünnungsmittel oder der Träger ein physiologisch annehmbarer fester Träger ist.

21. Pharmazeutisches Mittel nach Anspruch 19, wobei das Verdünnungsmittel oder der Träger eine physiologisch annehmbare Flüssigkeit ist.

22. Pharmazeutisches Mittel nach Anspruch 19, 20 oder 21, das zur oralen Verabreichung geeignet ist.

23. Pharmazeutisches Mittel nach Anspruch 19, 20 oder 21, das in injizierbarer Form oder in Form von Suppositorien vorliegt.

24. Pharmazeutisches Mittel, umfassend eine Verbindung der Formel (1) nach Anspruch 1, zusammen mit einem physiologisch annehmbaren Verdünnungsmittel oder Träger, das ein festes Mittel ist, geeignet zur oralen Verabreichung oder ein flüssiges injizierbares Mittel, das steril und pyrogenfrei ist.

25. Pharmazeutisches Mittel nach einem der Ansprüche 19 bis 24, in Form einer Einheitsdosis.

26. Pharmazeutisches Mittel nach einem der Ansprüche 19 bis 25, bei dem die Verbindung der Formel (1), wie in einem der Ansprüche 3 bis 18 definiert ist.

27. Verwendung einer Verbindung der Formel (1), wie in Anspruch 1 definiert, zur Herstellung eines Arzneimittels zur Verwendung zur Verringerung von toxischen Eisengehalten im Körper eines Patienten.

28. Verwendung nach Anspruch 27, wobei die Verbindung der Formel (1), wie in einem der Ansprüche 3 bis 18 definiert ist.

**Revendications**

1. Composé de formule (1)

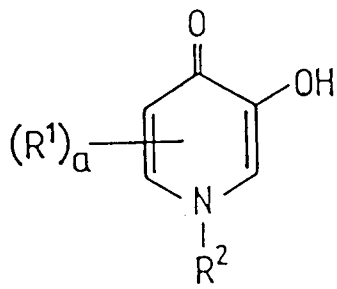

(1)

dans laquelle chaque $R^1$ séparément est un groupe hydrocarboné aliphatique en $C_1$ à $C_6$ ou un groupe hydrocarboné aliphatique en $C_1$ à $C_8$ substitué par un groupe formyloxy, (hydrocarbyl aliphatique en $C_1$ à $C_6$)-carbonyloxy, (hydrocarbyl aliphatique en $C_1$ à $C_6$)-oxycarbonyle, (hydrocarbyl aliphatique en $C_1$ à $C_6$)-sulfonyloxy, (hydrocarbyl aliphatique en $C_1$ à $C_6$)-oxysulfonyle, un groupe halogèno ou un groupe hydroxy,

$R^2$ est un groupe hydrocarboné aliphatique en $C_1$ à $C_6$, un groupe formyle ou (alkyl en $C_1$ à $C_4$)-carbonyle, ou un groupe hydrocarboné aliphatique en $C_1$ à $C_8$ substitué par un ou plusieurs substituants choisis parmi les groupes formyle, (alkyl en $C_1$ à $C_4$)-carbonyle, carbamoyle, sulfamoyle, carbamoyle à N et sulfamoyle à N mono- et disubstitués par un groupe hydrocarbyle aliphatique en $C_1$ à $C_6$, formylamino, (hydrocarbyl aliphatique en $C_1$ à $C_6$)-carbonylamino, (hydrocarbyl aliphatique en $C_1$ à $C_6$)-sulfonylamino, formylamino à N mono-substitué par un groupe hydrocarbyle aliphatique en $C_1$ à $C_6$, (hydrocarbyl aliphatique en $C_1$ à $C_6$)-carbonylamino N-substitué et (hydrocarbyl aliphatique en $C_1$ à $C_6$)-sulfonylamino N-substitué, formyloxy, (hydrocarbyl aliphatique en $C_1$ à $C_6$)-carbonyloxy, (hydrocarbyl aliphatique en $C_1$ à $C_6$)-oxycarbonyle, (hydrocarbyl aliphatique en $C_1$ à $C_6$)-sulfonyloxy, (hydrocarbyl aliphatique en $C_1$ à $C_6$)-oxysulfonyle, halogèno et hydroxy, et a est 0, 1, 2 ou 3, mais à l'exclusion des composés dans lesquels chaque $R^1$ et $R^2$ sont choisis parmi les groupes hydrocarbonés aliphatiques en $C_1$ à $C_6$ et de ceux dans lesquels le groupe >N-$R^2$ contient soit un groupe

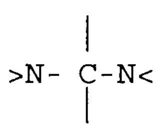

soit un groupe

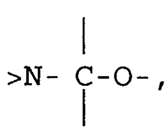

ou un sel physiologiquement acceptable de celui-ci, pour l'utilisation en thérapie.

**2.** Composé de formule (1)

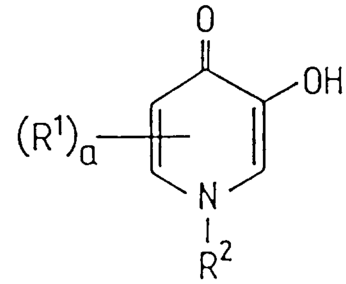

(1)

dans laquelle chaque $R^1$ séparément est un groupe hydrocarboné aliphatique en $C_1$ à $C_6$ ou un groupe hydrocarboné aliphatique en $C_1$ à $C_8$ substitué par un groupe formyloxy, (hydrocarbyl aliphatique en $C_1$ à $C_6$)-carbonyloxy, (hydrocarbyl aliphatique en $C_1$ à $C_6$)-oxycarbonyle, (hydrocarbyl aliphatique en $C_1$ à $C_6$)-sulfonyloxy, (hydrocarbyl aliphatique en $C_1$ à $C_6$)-oxysulfonyle, halogèno ou hydroxy,

$R^2$ est un groupe hydrocarboné aliphatique en $C_1$ à $C_6$, un groupe formyle ou (alkyl en $C_1$ à $C_4$)-carbonyle, ou un groupe hydrocarboné aliphatique en $C_1$ à $C_8$ substitué par un ou plusieurs substituants choisis parmi les groupes formyle, (alkyl en $C_1$ à $C_4$)-carbonyle, carbamoyle, sulfamoyle, carbamoyle à N et sulfamoyle à N mono- et disubstitués par un groupe hydrocarbyle aliphatique en $C_1$ à $C_6$, formylamino, (hydrocarbyl aliphatique en $C_1$ à $C_6$)-carbonylamino, (hydrocarbyl aliphatique en $C_1$ à $C_6$)-sulfonylamino, formylamino à N mono-substitué par un groupe hydrocarbyle aliphatique en $C_1$ à $C_6$, (hydrocarbyl aliphatique en $C_1$ à $C_6$)-carbonylamino N-substitué, et (hydrocarbyl aliphatique en $C_1$ à $C_6$)-sulfonylamino N-substitué, formyloxy, (hydrocarbyl aliphatique en $C_1$ à $C_6$)-carbonyloxy, (hydrocarbyl aliphatique en $C_1$ à $C_6$)-oxycarbonyle, (hydrocarbyl aliphatique en $C_1$ à $C_6$)-sulfonyloxy, (hydrocarbyl aliphatique en $C_1$ à $C_6$)-oxysulfonyle, halogèno et hydroxy, et a est 1, 2 ou 3 ;

mais à l'exclusion des composés dans lesquels chaque $R^1$ et $R^2$ sont choisis parmi les groupes hydrocarbonés aliphatiques en $C_1$ à $C_6$, des composés dans lesquels le groupe >N-$R^2$ contient soit un groupe

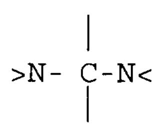

soit un groupe

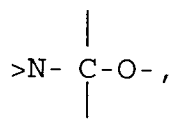

et des composés dans lesquels $(R^1)_a$ est un groupe 6-chlorométhyle et $R^2$ est un groupe méthyle, $(R^1)_a$ est un groupe 6-hydroxyméthyle et $R^2$ est un groupe méthyle ou éthyle, ou $(R^1)_a$ est un groupe 2-méthyle et $R^2$ est un groupe 2-hydroxyéthyle ou méthoxycarbonylméthyle ; ou un sel physiologiquement acceptable de celui-ci.

**3.** Composé selon la revendication 1 ou 2, dans lequel a est 0, 1 ou 2.

**4.** Composé selon la revendication 3, dans lequel a est 1 ou 2.

**5.** Composé selon l'une quelconque des revendications précédentes, qui a un groupe $R^1$ à la position 2.

**6.** Composé selon l'une quelconque des revendications précédentes, dans lequel $R^1$ est un groupe hydrocarboné aliphatique en $C_1$ à $C_4$ ou un groupe alkyle en $C_1$ à $C_3$ substitué par un groupe hydroxy.

7. Composé selon la revendication 4, dans lequel a est 1 et $R^1$ est un groupe alkyle en $C_1$ à $C_6$ à la position 2 ou à la position 6, ou bien a est 2 et $R^1$ est un groupe alkyle en $C_1$ à $C_6$ aux positions 2 et 6.

8. Composé selon la revendication 6 ou 7, dans lequel $R^1$ est un groupe méthyle, éthyle, propyle ou isopropyle.

9. Composé selon la revendication 4, dans lequel a est 1 et $R^1$ est un groupe méthyle ou éthyle à la position 2 ou un groupe méthyle à la position 6, ou bien a est 2 et $R^1$ est un groupe méthyle à chacune des positions 2 et 6.

10. Composé selon l'une quelconque des revendications précédentes, dans lequel $R^2$ est un groupe hydrocarboné aliphatique en $C_1$ à $C_6$.

11. Composé selon l'une quelconque des revendications précédentes, dans lequel un quelconque groupe hydrocarbyle dans le substituant d'un groupe hydrocarboné aliphatique $R^2$ substitué est un groupe en $C_1$ à $C_4$.

12. Composé selon l'une quelconque des revendications précédentes, dans lequel $R^2$ est un groupe $-(CH_2)_mOH$ dans lequel m est un nombre entier de 2 à 4.

13. Composé selon l'une quelconque des revendications 1 à 11, dans lequel $R^2$ est un groupe hydrocarboné aliphatique en $C_1$ à $C_6$ substitué par un groupe formyloxy, (alkyl en $C_1$ à $C_4$)-carbonyloxy, (alkyl en $C_1$ à $C_4$)-oxycarbonyle, (alkyl en $C_1$ à $C_4$)-sulfonyloxy ou (alkyl en $C_1$ à $C_4$)-oxysulfonyle.

14. Composé selon l'une quelconque des revendications 1 à 11, dans lequel $R^2$ est un groupe hydrocarboné aliphatique en $C_1$ à $C_6$ substitué par un groupe carbamoyle, sulfamoyle, carbamoyle à N ou sulfamoyle à N mono- ou disubstitué par un groupe alkyle en $C_1$ à $C_4$, formylamino, (alkyl en $C_1$ à $C_4$)-carbonylamino, (alkyl en $C_1$ à $C_4$)-sulfonylamino, formylamino à N mono-substitué par un groupe alkyle en $C_1$ à $C_4$, (alkyl en $C_1$ à $C_4$)-carbonylamino N-substitué ou (alkyl en $C_1$ à $C_4$)sulfonylamino N-substitué.

15. Composé selon l'une quelconque des revendications précédentes, dans lequel $R^2$ est un groupe alkyle en $C_1$ à $C_4$ substitué.

16. Composé selon l'une quelconque des revendications 1 à 9, dans lequel $R^2$ est un groupe alkyle en $C_1$ à $C_4$.

17. Composé selon la revendication 1, étant la 3-hydroxy-1-(2'-hydroxyéthyl)-2-méthylpyrid-4-one ou un sel physiologiquement acceptable de celle-ci.

18. Composé selon la revendication 1 ou 2, étant la 3-hydroxy-1-(3'-hydroxypropyl)-2-méthylpyrid-4-one ou un sel physiologiquement acceptable de celle-ci.

19. Composition pharmaceutique comprenant un composé selon la revendication 2, avec un diluant ou véhicule physiologiquement acceptable.

20. Composition pharmaceutique selon la revendication 19, dans laquelle le diluant ou véhicule est un véhicule solide physiologiquement acceptable.

21. Composition pharmaceutique selon la revendication 19, dans laquelle le diluant ou véhicule est un liquide physiologiquement acceptable.

22. Composition pharmaceutique selon la revendication 19, 20 ou 21, qui est adaptée pour l'administration par voie orale.

23. Composition pharmaceutique selon la revendication 19, 20 ou 21, qui est sous forme injectable ou sous forme de suppositoire.

24. Composition pharmaceutique comprenant un composé de formule (1) tel que défini dans la revendication 1, avec un diluant ou véhicule physiologiquement acceptable, qui est une composition solide adaptée pour l'administration par voie orale ou une composition liquide injectable qui est stérile et exempte de substance pyrogène.

25. Composition pharmaceutique selon l'une quelconque des revendications 19 à 24, sous forme galénique unitaire.

**26.** Composition pharmaceutique selon l'une quelconque des revendications 19 à 25, dans laquelle le composé de formule (1) est tel que défini dans l'une quelconque des revendications 3 à 18.

**27.** Utilisation d'un composé de formule (1) tel que défini dans la revendication 1, pour la fabrication d'un médicament pour utilisation en vue d'effectuer une réduction de taux toxiques de fer dans le corps d'un patient.

**28.** Utilisation selon la revendication 27, dans laquelle le composé de formule (1) est tel que défini dans l'une quelconque des revendications 3 à 18.